(19) Europäisches Patentamt
European Patent Office
Office européen des brevets

(11) **EP 4 785 860 A1**

(12) **EUROPEAN PATENT APPLICATION**

(43) Date of publication:
**05.08.2026 Bulletin 2026/32**

(21) Application number: **26152676.8**

(22) Date of filing: **19.01.2026**

(51) International Patent Classification (IPC):
**A61B 5/024** *(2006.01)*          **A61B 5/11** *(2006.01)*
**A61B 5/22** *(2006.01)*          **A61B 5/00** *(2006.01)*

(52) Cooperative Patent Classification (CPC):
**A61B 5/024;** A61B 5/1128; A61B 5/222;
A61B 5/486; A61B 5/742; A61B 5/744;
A61B 2505/09

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **24.01.2025 IT 202500001326**

(71) Applicant: **Technogym S.p.A.**
**47521 Cesena (FC) (IT)**

(72) Inventors:
• **ZOFFOLI, Luca**
  **47521 Cesena (FC) (IT)**
• **DI DONATO, Valentina**
  **47521 Cesena (FC) (IT)**

(74) Representative: **De Santis, Giovanni et al**
**c/o Giambrocono & C. S.p.A.**
**Via Rosolino Pilo, 19/B**
**20129 Milano (IT)**

(54) **MEASUREMENT STATION FOR ASSESSING THE CARDIORESPIRATORY FITNESS STATE OF A USER**

(57)     Measurement station (1) for assessing the cardiorespiratory fitness state of a user (2) by means of a predefined test to be performed, comprising:
- one display (3) and optionally a loudspeaker (4);
- a camera (5) for capturing images of the user;
- a controller (10) configured to process signals indicative of the heart rate of the user (2) received from a heart rate sensor (20) used by the user.

The controller presents during various phases of the test, through the display and/or said loudspeaker instructions which guide the user to assume a certain position, to calculate a first heart rate value, and to perform a predetermined number of repetitions of a predetermined exercise.

Moreover, in a final phase of the test, the controller calculates a second value and a third value of the heart rate of the user based on signals received by the sensor respectively during an initial time interval and a final time interval of such phase, and a parameter indicative of the cardiorespiratory fitness state of the user at least based on such first, second and third heart rate values.

*Fig. 1*

**Description**

The present invention is related in general to the wellness field.

In particular, the present invention is related to a measurement station for assessing the cardiorespiratory fitness state of a user (or person).

The present invention is particularly suitable for assessing the cardiorespiratory fitness state of users by evaluating of their VO2max.

As known, over the past few decades the so-called fitness world and then the conceptually broader wellness world has had a remarkable expansion.

Indeed, an increasingly large segment of the population has begun to dedicate more and more time to fitness exercise attending more or less regularly various types of gyms or fitness centers, also in old age.

Therefore, the industry professionals have progressively defined some reference parameters and they have developed various methodologies, equipment and machines suitable for measuring such parameters indicative of the fitness status. Among these, parameters related to the assessment of the cardiorespiratory fitness state assume particular relevance.

For example, a parameter used for assessing the cardiorespiratory fitness state is the so-called maximum oxygen uptake (VO2max), which essentially measures a user's maximum capacity to consume oxygen.

Practically, through such parameter there is an indication of the maximum exercise intensity that a subject can sustain through the aerobic metabolism for relatively long periods of time.

VO2max can be measured through the application of sophisticated and expensive equipment, e.g. metabolic carts, which require highly specialized personnel and that are available only in specific structures, e.g. sports medicine centers or anyway in specialized laboratories.

Alternatively, VO2max can be measured through methods and instruments accessible to everyone, e.g. using modern smartwatches which however provide quite approximate indications.

Therefore, the present invention aims to provide a solution which allows on one hand to provide a reasonably accurate assessment of the cardiorespiratory fitness state of a user, and in particular of the VO2max, and on the other hand to be easily accessible and user-friendly.

Such aim, and others which eventually result from the following description, is reached by a measurement station for the assessment of the cardiorespiratory fitness state of a user whose features are defined in claim 1.

Particular embodiments form object of the dependent claims, whose content is to be intended as an integral part of the present description.

Further features and advantages of the invention will become apparent in the following detailed description, provided purely by way of non-limiting example, with reference to the appended drawings, wherein:

figure 1 is a perspective view illustrating a possible embodiment of a measurement station configured for assessing the cardiorespiratory fitness state of a user, according to the present invention;

figure 2 schematically illustrates an image of a user on a display of the measurement station according to the invention, during a first phase of a predefined test which the measurement station is guiding to perform to measure a parameter indicative of the cardiorespiratory fitness state;

figures from 3 to 5 schematically illustrate some images in sequence on a display of the measurement station according to the invention, of a user during a second phase of the test that the measurement station is guiding to perform to measure a parameter indicative of the cardiorespiratory fitness state;

figures from 6 to 8 schematically illustrate some images in sequence on a display of the measurement station according to the invention, of a user during a second phase of the test that the measurement station is guiding to perform to measure a parameter indicative of the cardiorespiratory fitness state;

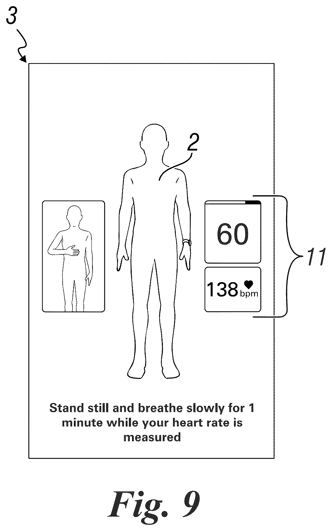

figure 9 schematically illustrates an image on a display of the measurement station according to the invention, of a user during a third phase of the test that the measurement station is guiding to perform to measure a parameter indicative of the cardiorespiratory fitness state.

It should be noted that in the detailed description that follows, in order to clearly and concisely describe the present invention, the drawings might not be necessarily on scale and some features of the description may be illustrated in a someway schematic form.

Moreover, when the term "configured", or "adapted", or "shaped", or "set", or a similar term is eventually used in the present document, with reference to any component as a whole, or to any part of a component or to a combination of components, it should be intended that it means and correspondingly comprises the structure and/or configuration and/or shape and/or positioning.

In particular, when such terms refer to hardware or software electronic means, they should be intended as including circuits or parts of electronic circuits, as well as software/firmware, such as algorithms, routines and programs in general, under execution and/or stored on any type of storage medium.

Lastly, when the term "substantial" or "substantially" is used herein, it should be intended as an actual variation of more or less the 5% compared to what is indicated as reference value or position.

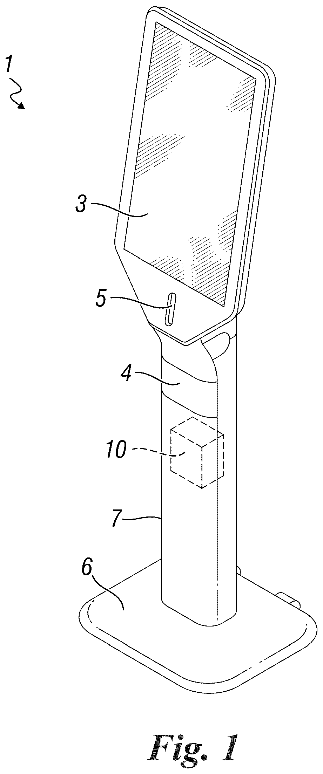

Figure 1 is a perspective view illustrating a possible embodiment of a measurement station configured to measure a parameter indicative of the cardiorespiratory fitness state according to the present invention, indicated by the overall reference number 1.

In a possible embodiment, the measurement station 1 according to the present invention is configured to measure a value indicative of the VO2max of a user.

The definition of measurement station 1 is hereby to be intended as including both an apparatus made in a single structure (as illustrated in Figure 1) which integrates various constitutive components/parts, and possibly an apparatus made by two or more separated parts/devices and connected between them.

Moreover, in the following description, reference will be made to the measurement station 1 as configured to make a user, whose parameter indicative of cardiorespiratory fitness state, in particular the VO2max, is to be evaluated, to perform a predefined test which in particular involves the performance of an exercise composed by a series of squats, e.g. in a specific time interval.

Clearly, such exercise should be intended by way of non-limiting example since, for the assessment of the cardiorespiratory fitness state, e.g. VO2max, the measurement station 1 according to the invention may be configured to make the user perform one or more other types of exercises.

Usefully, the measurement station 1 according to the invention comprises:

- at least one display or touchscreen 3 (in the following display 3) and optionally a loudspeaker 4;
- at least one controller or one electronic control unit (in the following controller), schematically illustrated in Figure 1 by the dashed box 10, configured to process signals indicative of the heart rate of the user 2 received by a heart rate sensor 20 used by the user 2 themselves during the performance of the predefined test;
- a camera 5 configured to capture and send to the controller 10 one or more images of the user 2 when executing said predefined test.

Clearly, as evident for a person skilled in the art, the definition of loudspeaker 4 as optional element of the measurement station 1 is justified by the fact that:

- the loudspeaker 4 may be used and some of the messages and/or instructions for the performance of the test may be conveyed to the user in audio form, in addition or alternatively with respect to what is graphically shown on the display 3; or
- the loudspeaker may be omitted, or however not used for the aims and functionalities of the present invention, and therefore the messages/instructions are all conveyed through the display 3.

Moreover, during the performance of the test, the instructions emitted on the display 3 and/or through the loudspeaker 4, may be accompanied by the projection of a reference image on a window of the display 3 itself, as illustrated for the sake of descriptive simplicity only in Figure 9.

For example, in the embodiment illustrated in Figure 1, the measurement station 1 comprises at least a lying base 6, a support 7 which protrudes from the lying base 6 along which the loudspeaker 4 is placed, wherever used.

The display 3 is for example placed on the end of the support 7 opposite to the lying base 6.

For example, the camera 5 is placed at the basis of the display 3.

The camera 5 can be for example a digital 2D camera, a digital 3D camera, an RGB camera, an RGB-D camera, a video camera, a depth camera, a Time-Of-Flight (TOF) camera, a webcam, thermal imaging camera, et cetera.

The heart rate sensor 20 can be constituted by any device suitable for the use and capable of being placed in communication with and transmit signals to said controller 10, e.g. a wrist heart rate monitor, a chest-strap heart rate monitor, a wearable device as e.g. a smartwatch, a device with detecting systems based on optical cameras, etc.

The controller 10 can be formed or can comprise an electronic control unit which includes, for example, a processor, one or more software modules or algorithms which, once executed by the processor, allow to perform the various expected functionalities for the controller 10, one or more communication modules for the reception and/or transmission of data/signals with the external environment, for example with a remote controller and with other devices.

The controller 10 can also comprise for example one or more data storage units for storing data locally.

The display or touchscreen 3 is provided for example with a graphical interface which presents to the user one or more guiding windows both to start the performance of the predefined test, and for other reasons.

For example, before performing the predefined test, a user 2 interacts or connects directly to the measurement station 1 to enter at least some parameters relevant to the aim of the calculation of a parameter indicative of their cardiorespiratory fitness state, e.g. the calculation of a value indicative of their VO2max, or to possibly access to their data whether already existent by controlling them and editing them just in case.

For example, the access to the measurement station 1 can happen directly through the graphical interface of the display 3, by entering or creating their own user identification data, e.g. username and password, or through a smartphone which reads a QR code on the display 3.

The data/parameters of the user can be saved both on a local storage unit part of or in any case accessible by the controller 10, or they can be recorded remotely, e.g. in the cloud, and made available to the controller itself 10 also in this case.

In particular, in a possible embodiment, to the aim of calculating the VO2max through the measurement station 1 according to the invention, the user 2 should enter or ensure themselves that data related at least to their age, gender and height are recorded and/or updated.

In the case of indeterminacy or non-specification of their own gender, in a possible embodiment, the measurement station 1 is configured to automatically assign to the user 2 the female category.

Alternatively, some data related to the user can be detected through a camera which captures one or more images of the user themselves.

The captured images are received and processed by the controller 10 in order to derive age, gender, height and possibly the weight of the user.

Clearly, as a function of the applications, and in particular of the type of the predefined test that is intended to be performed and/or of the formula/formulae used for the calculation of a parameter indicative of the cardiorespiratory fitness, e.g. VO2max, it is possible that the user 2 will be required to necessarily enter other data, e.g. their weight; moreover, it is possible to provide to the user 2 the possibility to enter/edit other data possibly relevant for this aim.

In a possible embodiment, in a phase prior to the performance of the predefined test, the controller 10 is configured to present on the display 3 a series of types/brands of heart rate sensors 20 so that the user 2 can select the one currently in use or a similar device.

In a possible embodiment, in a phase prior to the performance of the predefined test, the controller 10 is configured to verify the accuracy of the connection with and of the transmission of signals from the heart rate sensor 20 used by the user 2 and possibly indicate, through a text message showed on the display 3 and/or through an audio message conveyed through the loudspeaker 4, that the connection is correctly established and so that the predefined test can be started, or not.

In particular, in a possible embodiment, in a phase prior to the performance of the predefined test, the controller 10 detects through the camera 5 the image of the user 2 and it schematizes their body by generating a "skeleton" formed by a structure with segments which connect key points (nodes) placed in relevant body parts, such as joints or other important points, e.g. shoulders, knees, elbows, lower limbs and upper limbs extremities, etc. through computer vision techniques - pose estimation (e.g. one or more modules or algorithms of images/movements analysis).

On the basis of the images received from the camera and processed by the controller 10, the nodes extracted from the processed images are compared with the reference nodes, and the data are consequently processed.

From this the controller 10 derives the necessary information to define the minimum Range of Motion that the user 2 should move through to perform an exercise or a valid exercise repetition, e.g. one squat.

Conveniently, in the measurement station 1 according to the invention, the controller 10 is further configured to present through the display 3 and/or the loudspeaker 4:

- during a first phase of the predefined test, one or more first instructions suitable for guiding the user 2 to locate themselves at a determined distance from the camera 5, in such first phase of the predefined test the controller 10 calculating a first value HR1 of heart rate of the user 2 on the basis of the signals received from the heart rate sensor 4 during a first predefined time interval;

- during a second phase of the predefined test, one or more second instructions suitable for guiding the user 2 to perform a predetermined number of repetitions of a predetermined exercise, during said second phase the controller 10 being configured to present on the display 3 at least one target position that the user 2 has to reach in each repetition of the predetermined exercise in order for each performed repetition to be considered valid.

Usefully, the controller 10 is further configured to calculate, in a third phase of the predefined test, a second heart rate value HR2 and a third heart rate value HR3 of the user 2 on the basis of signals received from the heart rate sensor 20, respectively during a predetermined initial time interval and a predetermined final time interval of said third phase; and wherein the controller 10 is further configured to calculate one parameter indicative of the cardiorespiratory fitness state of the user at least on the basis of said calculated first, second and third heart rate values HR1, HR2, HR3 of the user 2.

In a possible embodiment, the controller 10 is configured to calculate said parameter indicative of the cardiorespiratory fitness state of the user 2 at least on the basis of said calculated first, second and third heart rate values of the user 2 and on one or more prerecorded data related to the user 2 themselves.

In particular, in a possible embodiment, the controller 10 is configured to calculate a value indicative of the VO2max of the user 2.

According to the applications, such value indicative of the VO2max can be calculated only as a function of the calculated first, second and third heart rate values HR1, HR2 and HR3 of the user 2 or also on the basis of said one or more prerecorded data related to the user 2 themselves.

In a possible embodiment, the controller 10 is configured to present through the display 3 and/or the loudspeaker 4, during said first phase of the predefined test, one or more first instructions suitable for guiding the user 2 to position themselves at a determined distance from the camera 5 and to assume a determined pose or posture.

In a possible embodiment, during such second phase, the user 2 can have only one objective of fulfilment of a predetermined number of repetitions, and subsequently the controller 10 verifies the time that it took and, on the basis of a maximum time limit, it evaluates whether the second phase is to be considered as completed, or whether the test has to be repeated from the beginning.

In a possible embodiment, the controller 10 is configured to present, through said display 3 and/or said loudspeaker 4, during the second phase of the predefined test, one or more second instructions suitable for guiding the user 2 to perform a predetermined number of repetitions of a predetermined exercise, in a second predefined time interval.

In a possible embodiment, the controller 10 is configured to present, through said display 3 and/or said loudspeaker 4, during the third phase of the predefined test, one or more third instructions suitable for guiding the user 2 to assume a determined position.

In a possible embodiment, the controller 10 is for example configured to calculate a parameter indicative of the cardiorespiratory fitness state of the user 2 on the basis of one of the following formulae known in scientific literature:

- *Ruffier Index:*

$$RI = \frac{(HR1+HR2+HR3-200)}{10}$$

- *Ruffier-Dickinson Index:*

$$RDI = \frac{(HR2-70)+2*(HR3-HR1)}{10}$$

In a possible embodiment, the controller 10 is for example configured to calculate a value indicative of the VO2max on the basis of the following formula used in scientific literature (see, for example Guo Y, Bian J, Li Q, Leavitt T, Rosenberg EI, et al. (2018), A 3-minute test of cardiorespiratory fitness for use in primary care clinics. PLOS ONE 13(7): e0201598):

*VO2max (l/min) =+3.0143 + (1.1585 * (0, Female, 1 Male) -0.0268 * HR1/Height (m) + 118.7611 * (HR2 - HR3)/Age³*

Alternatively, in case of other parameters of the user 2, e.g. weight, are kept into consideration, the controller 10 is for example configured to calculate the VO2max on the basis of the following formula, wherein the VO2max (l/min) can be calculated with the previous formula:

*VO2max (ml/kg/min) =1000 / Weight (kg) * VO2max (l/min)*

In a possible embodiment, the measurement station 1 is configured to show to the user 2 on the display 3 a video tutorial and/or a message indicative of the type and/or the number of repetitions to be performed, e.g. thirty squats.

In the case where also the second time interval during which the predetermined number of exercises have to be performed is set, which can be for example a few tens of seconds, e.g. 45 seconds, also that indication is provided to the user 2.

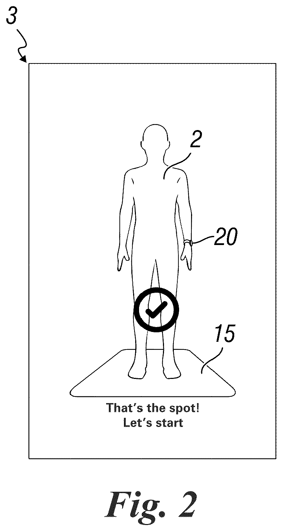

For example, as schematically illustrated in Figure 2, in the first phase of the test, the camera 5 captures one or more images of the user 2 which are sent to and processed by the controller 10.

In a possible embodiment, the controller 10, through the processing of one or more images captured by the camera 5, checks that the user 2 has assumed a position suitable for performing a predetermined exercise.

For example, in the case wherein the predetermined exercised is related to the performance of a defined number of squats in a predetermined time, the controller 10 checks that the user 2 is in a standing position with the feet distant from each other by a distance which is substantially equal to the width of their shoulders.

In a possible embodiment, and as shown in Figure 2, an area 15, correspondent to a distance from the display 3 (or from another reference point of the station 1) wherein the user 2 has to place themselves, is visualized on the display 3.

Therefore, the controller 10 checks that the user 2 is in a standing position with the feet distant from each other by a distance substantially equal to the width of their shoulders, inside the area 15.

Once that each predetermined condition is satisfied, in a possible embodiment, the controller 10 presents a message to the user 2, via the display 3 and/or the loudspeaker 4, indicative of the correctness of the position assumed and that the test can proceed by calculating the first value of heart rate.

In particular, in a possible embodiment, in such first phase, the controller is configured to calculate the first heart rate value HR1 of the user 2 on the basis of the signals received from the heart rate sensor 20 only if at least a predetermined condition is fulfilled during said first predefined time interval.

In the case where said predetermined condition is not fulfilled, the controller 10 is configured to provide to the user 2, through the display 3 and/or the loudspeaker 4, a message indicating that the first predefined time interval is extended with additional time when such predetermined condition is not fulfilled, and to abort the performance of the test when such predetermined condition is not fulfilled also at the expiration of additional time.

For example, the first time interval can be equal to a few tens of seconds, e.g. 50 seconds.

In a possible embodiment, the controller 10 is configured to provide to the user 2, through the display 3 and/or the loudspeaker 4, a message of maintaining the assumed standing position and breathing slowly ("Stand still and breathe slowly while your resting heart rate is measured").

If in such first time interval, or at least in a subpart of it, the heart rate is substantially stable, i.e. the number of heartbeats detected by the sensor 20 do not differ from each other for a number higher than a predetermined threshold, then the value HR1 is calculated and it is equal to the average of the measured values in such first interval, or at least in a subpart of it.

When the heart rate is unstable, i.e. the values of the detected heartbeats differ by a number higher of the predetermined threshold, the first time interval is extended by the controller 10 of such additional time, for example until two minutes.

In such circumstance, in a possible embodiment, the controller 10 is configured to provide to the user 2, through the display 3 and/or the loudspeaker 4, a message indicative of the fact that the heart rate is unstable and additional time is added.

If also at the end of the additional time the heart rate is still unstable, in a possible embodiment, the controller 10 is configured to abort the performance of the test.

In such circumstance, in a possible embodiment, the controller 10 is configured to provide to the user 2, through the display 3 and/or the loudspeaker 4, a message indicative of the fact that, due to the lasting instability of the heart rate, the test has to be suspended, for example starting again in a following time.

In a possible embodiment, the controller 10 is configured to visualize in this phase on the display 3 a timer 11 to notify the user 2 about the remaining time and that the measurement is taking place.

In a possible embodiment, the controller is configured to consider valid a repetition of the predetermined exercise when the user 2 reached said at least a target position on the basis of one or more images captured and received by the camera 5 during the performance of each repetition.

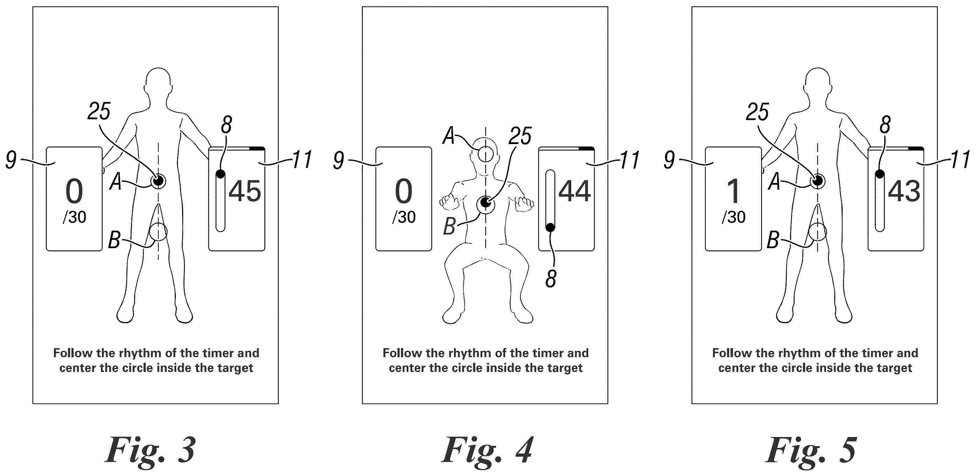

In a possible embodiment, the controller 10 is configured to present on the display 3 during said second phase of the predefined test, a first target position (indicated in Figures 3-8 by letter A) and a second target position (indicated in Figures 3-8 by letter B) that the user 2 has to reach in each repetition of the predetermined exercise, in order for each performed repetition to be considered valid.

For example, at the beginning of the second phase, the controller 10 is configured to calculate and visualize on the display 3 a first target position A at a reference point of the user 2 that is being tested, e.g. in the middle point between the two hips. In an embodiment, the controller 10 is configured to calculate and visualize on the display 3 a second target position B along a line connecting the first target position A and the middle point between the two knees of the user.

For example, the target position B can be chosen at the 75% of the length of the segment connecting the middle point between the two hips with the middle point between the two knees.

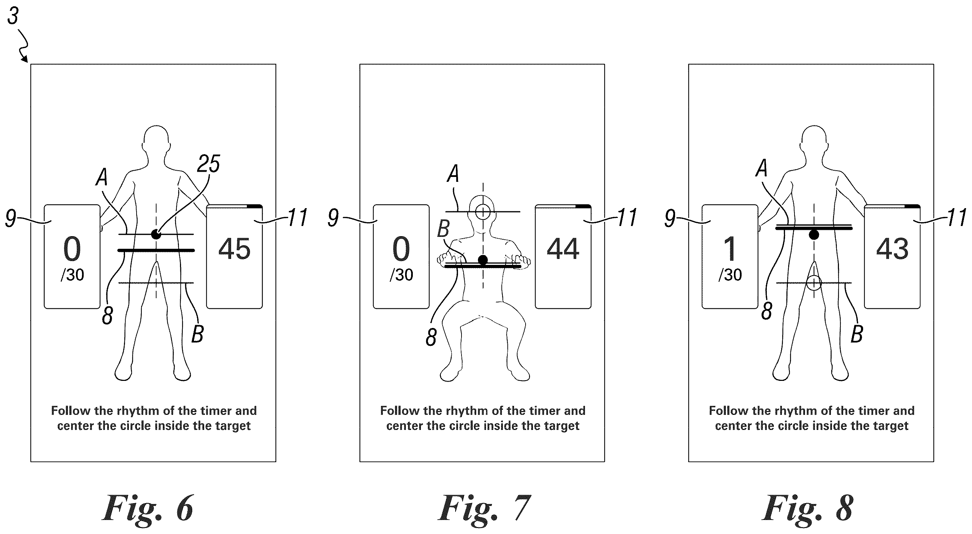

In particular, the controller 10 is configured to consider valid each repetition of the predetermined exercise when the user 2, starting from the first target position A (Figures 3 and 6), reached the second target position B (Figures 4 and 7) and came back reaching again the first position A (Figures 5 and 8), on the basis of and by electronically processing the one or more images captured and received from the camera 5 during the performance of each repetition.

Usefully, in a possible embodiment of the measurement station 1 according to the invention, the controller 10 is configured to present on the display 3, at least during the second phase of the predefined test, a reference indicator, represented in Figures from 3 to 8 by a circle 25, which is associated to a portion of the body of the user 2.

Moreover, in this embodiment, the controller 10 is configured to visualize on the display 3, on the basis of the received and processed one or more images captured by the camera 5, the reference indicator 25 moving together with the movement of the user 2 during the performance of the predetermined exercise in order to graphically indicate real-time the position reached by the user with respect to the at least one target position or to said first and second target positions A and B to be reached.

In such an embodiment, the two target positions A and B are represented by two circumferences inside which the reference circle 25 has to be substantially placed in order for the target position to be considered as reached.

For example, at the beginning of the second phase, the controller 10 is configured to calculate the first target position at a reference point of the user 2 who is being tested, for example in the middle point between the two hips.

For example, such condition whether successfully reached can be graphically highlighted on the display 3, e.g. by coloring each target with green, for example. In a possible embodiment, the controller 10 is configured to present on the display 3, at least during said second phase of the predefined test, a pacer 8 suitable for indicating to the user 2 a target cadence to be respected to perform the predetermined number of repetitions to be completed, for example in the second predefined time interval when imposed.

For example, in a possible embodiment schematically illustrated in Figures from 3 to 5, the pacer 8 is represented by a circle sliding in a slot 9 which has a predetermined height corresponding or proportional to the Range of Motion which the user 2 has to perform, calculated by the controller 10 on the basis of the anthropometric dimensions of the user 2 themselves detected by the images of the camera 5.

In another possible embodiment, schematically illustrated in Figures from 6 to 8, the pacer 8 is represented by a horizontal bar to be "chased" by the user 2 through the reference indicator 25 associated and moving together with the user themselves. In practice, during the performance of every repetition, i.e. in the example illustrated of a squat, the circle 25, which graphically represents on the display 3 the movement of the user 2, has to substantially overlap the bar 8. In this case, the pacer 8, represented by the horizontal bar to be "chased", has a descending vertical movement (from the top to the bottom) substantially coinciding with the Range of Motion that the user 2 has to perform in that direction; the same happens when the user must ascend to return to the starting position.

Such Range of Motion is calculated by the controller 10 for example on the basis of the anthropometric dimensions of the user 2 themselves detected by means of the images captured by the camera 5 and processed by the controller 10 itself.

In this embodiment, the target positions A and B can be represented only by the related circumferences, as illustrated in the Figures from 3 to 6, or the two circumferences can be associated to two correspondent horizontal lines (as illustrated in Figures from 6 to 8), or each one may be represented solely by a corresponding line.

Also in this case, the correct reaching of a reached target position can be graphically highlighted on the display 3, e.g. by coloring each target with green, for example. For example, the pacer 8 can be set at a cadence or frequency of some tens of bpm, e.g. 40 bpm.

In a possible embodiment, the controller 10 is configured to present on the display 3, at least during said second phase of the predefined test, at least one counter, indicated in Figures from 3 to 8 by the reference number 9, suitable for indicating to the user 2 the number of performed repetitions considered valid and possibly the time past from the beginning of the second phase and/or the time left with respect to the end of the second predefined time interval when imposed.

In a possible embodiment, the controller 10 is configured to track, for example as a function of the time past from the beginning of the second phase, or of the second predefined time interval when imposed, the number of repetitions (e.g. squats) performed and considered valid with respect to a number of expected repetitions, and to present to the user 2 through the display 3 and/or the loudspeaker 4, a message suitable for indicating to the user 2 a modification of the current cadence of the performance of repetitions when the number of performed repetitions considered valid differs from the expected number of repetitions.

For example, to this aim the controller 10 can use the following formula, in the case where, for example, the cadence is 40 bpm:

$$\textit{Number of expected repetitions = time past from the beginning of the exercise:1.5}$$

In this case, if the number of the detected repetitions is lower or higher with respect to the one expected, the controller 10 can convey to the user 2, through the display 3 and/or the loudspeaker 4, a message encouraging them to increase or respectively decrease the cadence.

Clearly, the second phase is completed when the user performed correctly the number of repetitions required, e.g. squats, in an acceptable time or in a time equal to at most the second predefined time interval when imposed.

At the beginning of the third phase, in a possible embodiment, the controller 10 can be configured to present to the user, through the display 3 and/or the loudspeaker 4, a message indicative of the position to be maintained in that phase for a specific time interval, e.g. one minute.

Whenever defined, said position to be maintained in the third phase is for example a position similar to the one to be maintained in the first phase.

Moreover, the controller 10 is for example configured to present to the user 2, through the display 3 and/or the loudspeaker 4, through the same message or a further message, the indication of breathing slowly.

In this third phase, on the basis of the signals received by the sensor 20, the controller 10 is configured to calculate the second value of heart rate HR2 and the third value of heart rate HR3.

For example, the second value HR2 is calculated as the average of the heart rates detected in a first time subinterval counted starting from the end of the second phase; for example, such first subinterval is comprised between 10 seconds and 15 seconds starting from the end of the second phase.

In turn, the third value HR3 is calculated as the average of the heart rates detected in a second time subinterval counted starting from the end of the second phase, but not immediately following the first subinterval; for example, such subinterval is comprised between 55 seconds and one minute starting from the end of the second phase.

In a possible embodiment, the controller 10 is configured to track, on the basis of one or more images received from the camera 5 during one or more of said first, second and third phases (and processed by the controller 10), the position of the user 2 and to convey to them, through the display 3 and/or the loudspeaker 4, an alert message or to abort the test whether the user moved from the predetermined distance (whenever imposed) or from the predetermined target position of a quantity higher than a predetermined threshold.

In a possible embodiment, the controller 10 is configured to present on the display 3, during one or more of the first, second and third phases, a timer 11 suitable for notifying the user 2 the time elapsed from the beginning of the corresponding first phase, second phase and third phase and/or the current heart rate.

It has been practically evidenced how the measurement station 1 according to the invention allows the intended purpose to be fulfilled since it is easily accessible and user-friendly and it allows calculating a parameter indicative of the cardiorespiratory fitness state of a user, for example the value of their VO2max, with a reasonably appropriate accuracy.

Obviously, without departing from the principle of the invention, the actuation embodiments and particulars may be widely varied with respect to what has been described and illustrated by way of non-limiting example, without thereby departing from the scope of protection of the present invention as defined in the appended claims; for example, the target positions indicated above may be differently located or defined.

**Claims**

1. Measurement station (1) for assessing the cardiorespiratory fitness state of a user (2) by means of a predefined test to be performed by the user (2), **characterized by** comprising:

   - at least one display (3) and optionally a loudspeaker (4);
   - a camera (5) configured to capture one or more images of the user (2);
   - a controller (10) configured to process signals indicative of the heart rate of the user (2) received from a heart rate sensor (20) used by the user (2) while performing said predefined test; wherein said controller (10) is further configured to present through said display (3) and/or said loudspeaker (4):
   - during a first phase of the predefined test, one or more first instructions designed to guide the user (2) to assume a certain position, in said first phase of the predefined test the controller (10) calculating a first heart rate value of the user (2) based on signals received from said heart rate sensor (20) during a first predefined time interval;
   - during a second phase of the predefined test, one or more second instructions designed to guide the user (2) to perform a predetermined number of repetitions of a predetermined exercise, during said second phase the controller (10) being configured to present on the display (3) at least one target position that the user (2) must reach in each repetition of the predetermined exercise for each repetition performed to be considered valid; wherein the controller (10) is further configured to calculate, during a third phase of the predefined test, a second value and a third value of the heart rate (2) of the user (2) based on signals received from said heart rate sensor (20) during an initial predefined time interval and a final predefined time interval of said third phase, respectively; and wherein said controller (10) is further configured to calculate a parameter indicative of the cardiorespiratory fitness state of the user (2) at least on the basis of said calculated first, second and third heart rate values of the user (2).

2. Measurement station (1) according to claim 1, wherein said controller (10) is configured to calculate said parameter indicative of the cardiorespiratory fitness state of the user (2) at least on the basis of said calculated first, second and third heart rate values of the user (2) and one or more prerecorded data related to said user (2).

3. Measurement station (1) according to claim 1, wherein said controller (10) is configured to calculate a value indicative of the VO2max of the user (2).

4. Measurement station (1) according to one or more of the preceding claims, wherein said controller (10) is configured to present on the display (3), during said second phase of the predefined test, a first target position (A) and a second target position (B) that the user (2) must reach in each repetition of the predetermined exercise, for each repetition performed to be considered valid.

5. Measurement station (1) according to claim 4, wherein said controller (10) is configured to consider valid each repetition of the predefined exercise when the user (2) starting from the first target position (A) reached the second target position (B) and returned reaching the first target position (A) again, based on one or more images captured by and received from the camera (5) during the execution of each repetition.

6. Measurement station (1) according to one or more of the preceding claims, wherein said controller (10) is configured to present on the display (3), at least during said second phase of the predefined test, a reference indicator (25) associated with a portion of the body of the user (2), said controller (10) being configured to display on the display (3), based on one or more images captured by the camera (5), the reference indicator (25) moving simultaneously with the movement of the user (2) during the execution of the predefined exercise, so as to graphically indicate in real-time the position reached by the user (2) with respect to the at least one target position or to said first and second target positions to be reached (A, B).

7. Measurement station (1) according to one or more of the preceding claims, wherein said controller (10) is configured to present on the display (3), at least during said second phase of the predefined test, a pacer (8) suitable for indicating to the user (2) a reference cadence to be observed for performing the predetermined number of repetitions to be performed in the second predefined time interval.

8. Measurement station (1) according to one or more of the preceding claims, wherein said controller (10) is configured to present on the display (3), at least during said second phase of the predefined test, at least one counter (9) adapted to present to the user (2) the number of executed repetitions deemed valid and the time remaining with respect to the end of the second predefined time interval.

9. Measurement station (1) according to one or more of the preceding claims, wherein said controller (10) is configured to monitor, as a function of the time elapsed from the beginning of said second phase or a second predefined time interval, the number of executed repetitions considered valid with respect to an expected number of repetitions, and to present to the user (2), through said display (3) and/or said loudspeaker (4), a message designed to indicate to the user (2) a change in the current cadence of execution of repetitions when the number of executed repetitions considered valid is different from the expected number of repetitions.

10. Measurement station (1) according to one or more of the preceding claims, wherein said controller (10) is configured to monitor, based on one or more images captured by the camera (5) during one or more of said first, second and third phases, the position of the user (2) and to present, via said display (3) and/or said loudspeaker (4), an alert message or abort the test if the user has moved from said determined position or from a predetermined reference position or distance by a respective amount greater than a corresponding predetermined threshold.

11. Measurement station (1) according to one or more of the preceding claims, wherein said controller (10) is configured to present on the display (3), during one or more of the first, second and third phases, a timer (11) suitable for indicating to the user (2) the time elapsed from the beginning of the respective first phase, second phase and third phase.

12. Measurement station (1) according to one or more of the preceding claims, wherein, in said first phase, said controller (10) is configured to calculate said first heart rate value of the user (2) based on signals received from said heart rate sensor (20) only if at least one predetermined condition is met during said first predefined time interval, and to send to the user (2), through said display (3) and/or said loudspeaker (4), a message indicating that said first predefined time interval is extended by an additional time when said predetermined condition is not met, and to abort performing the test if said predetermined condition is not met even after said additional time has expired.

**Fig. 1**

*Fig. 2*

**Fig. 3**

Follow the rhythm of the timer and
center the circle inside the target

**Fig. 4**

Follow the rhythm of the timer and
center the circle inside the target

**Fig. 5**

Follow the rhythm of the timer and
center the circle inside the target

**Fig. 6**

**Fig. 7**

**Fig. 8**

*Fig. 9*

| | Europäisches Patentamt European Patent Office Office européen des brevets | **EUROPEAN SEARCH REPORT** | Application Number EP 26 15 2676 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TROVATO BRUNO ET AL: "Modified Isoinertial-Based Ruffier Test in Healthy Individuals: A Feasibility Study", DEPARTMENT OF BIOLOGY, SBARRO INSTITUTE FOR CANCER RESEARCH AND MOLECULAR MEDICINE, COLLEGE OF SCIENCE AND TECHNOLOGY, TEMPLE UNIVERSITY, PHILADELPHIA, PA 19122, USA, vol. 8, no. 2, 24 March 2023 (2023-03-24), page 36, XP093303177, ISSN: 2411-5142, DOI: 10.3390/jfmk8020036 | 1-4,11 | INV. A61B5/024 ADD. A61B5/11 A61B5/22 A61B5/00 |
| Y | * the whole document * ----- | 5-10,12 | |
| Y | US 2021/008413 A1 (ASIKAINEN SAMI [CA] ET AL) 14 January 2021 (2021-01-14) * paragraphs [0046], [0066] - [0093]; figures 1B,4-6,10 * ----- | 5-10 | |
| Y | US 2023/094802 A1 (PUTNAM BRYNN [US] ET AL) 30 March 2023 (2023-03-30) * paragraphs [0173] - [0183]; claim 1; figure 2C * ----- | 5-10 | TECHNICAL FIELDS SEARCHED (IPC) |
| Y | US 2024/233443 A1 (RUSH DARREN [US] ET AL) 11 July 2024 (2024-07-11) * paragraphs [0059] - [0065]; figures 8,10 * ----- | 5-10 | A61B |
| Y | US 2024/153611 A1 (ARAGONES TERESA [US] ET AL) 9 May 2024 (2024-05-09) * claims 1,4; figures 9-19 * ----- | 5-10 | |
| Y | US 2022/072383 A1 (LIAO HUNG-MAO [TW] ET AL) 10 March 2022 (2022-03-10) * paragraphs [0033] - [0038]; claim 1; figures 4-5 * ----- -/-- | 5-10 | |

The present search report has been drawn up for all claims

| Place of search | Date of completion of the search | Examiner |
|---|---|---|
| Berlin | 8 June 2026 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 26 15 2676

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2001 218745 A (NAGANO NATIONL COLLEGE OF TECH) 14 August 2001 (2001-08-14) * paragraphs [0020] - [0021] * ----- | 12 | |
| A | US 2024/342553 A1 (ZOFFOLI LUCA [IT] ET AL) 17 October 2024 (2024-10-17) * paragraphs [0008], [0110] * ----- | 1 | |
| X,D | GUO YBIAN JLI QLEAVITT TROSENBERG EL ET AL.: "A 3-minute test of cardiorespiratory fitness for use in primary care clinics.", PLOS ONE, vol. 13, no. 7, 2018, page e0201598, XP093302708, * the whole document * ----- | 1-3 | |
| | | | TECHNICAL FIELDS SEARCHED (IPC) |

The present search report has been drawn up for all claims

| Place of search | Date of completion of the search | Examiner |
|---|---|---|
| Berlin | 8 June 2026 | Kronberger, Raphael |

EPO FORM 1503 03.82 (P04C01)

page 2 of 2

## ANNEX TO THE EUROPEAN SEARCH REPORT
## ON EUROPEAN PATENT APPLICATION NO.

EP 26 15 2676

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2026

| Patent document cited in search report | | Publication date | Patent family member(s) | | Publication date |
|---|---|---|---|---|---|
| US 2021008413 | A1 | 14-01-2021 | CA | 3146658 A1 | 14-01-2021 |
| | | | EP | 3996822 A1 | 18-05-2022 |
| | | | GB | 2600289 A | 27-04-2022 |
| | | | US | 2021008413 A1 | 14-01-2021 |
| | | | WO | 2021007581 A1 | 14-01-2021 |
| US 2023094802 | A1 | 30-03-2023 | CA | 3176608 A1 | 04-11-2021 |
| | | | CN | 115485753 A | 16-12-2022 |
| | | | EP | 4143809 A1 | 08-03-2023 |
| | | | KR | 20230003110 A | 05-01-2023 |
| | | | US | 2021339110 A1 | 04-11-2021 |
| | | | US | 2021342952 A1 | 04-11-2021 |
| | | | US | 2022387874 A1 | 08-12-2022 |
| | | | US | 2023077227 A1 | 09-03-2023 |
| | | | US | 2023094802 A1 | 30-03-2023 |
| | | | WO | 2021222497 A1 | 04-11-2021 |
| US 2024233443 | A1 | 11-07-2024 | US | 2024233443 A1 | 11-07-2024 |
| | | | WO | 2022221685 A1 | 20-10-2022 |
| US 2024153611 | A1 | 09-05-2024 | BR | 112013011083 A2 | 23-08-2016 |
| | | | CA | 2816589 A1 | 10-05-2012 |
| | | | CN | 103282907 A | 04-09-2013 |
| | | | EP | 2635988 A1 | 11-09-2013 |
| | | | JP | 6077683 B2 | 08-02-2017 |
| | | | JP | 6307183 B2 | 04-04-2018 |
| | | | JP | 2014502178 A | 30-01-2014 |
| | | | JP | 2016120297 A | 07-07-2016 |
| | | | JP | 2017060891 A | 30-03-2017 |
| | | | KR | 20130116886 A | 24-10-2013 |
| | | | US | 2012183940 A1 | 19-07-2012 |
| | | | US | 2016279475 A1 | 29-09-2016 |
| | | | US | 2020147454 A1 | 14-05-2020 |
| | | | US | 2021358591 A1 | 18-11-2021 |
| | | | US | 2024153611 A1 | 09-05-2024 |
| | | | US | 20260038665 A1 | 05-02-2026 |
| | | | WO | 2012061804 A1 | 10-05-2012 |
| US 2022072383 | A1 | 10-03-2022 | CN | 114237777 A | 25-03-2022 |
| | | | US | 2022072383 A1 | 10-03-2022 |
| JP 2001218745 | A | 14-08-2001 | JP | 3421738 B2 | 30-06-2003 |
| | | | JP | 2001218745 A | 14-08-2001 |
| US 2024342553 | A1 | 17-10-2024 | EP | 4445839 A1 | 16-10-2024 |
| | | | US | 2024342553 A1 | 17-10-2024 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 26 15 2676

This annex lists the patent family members relating to the patent documents cited in  the above-mentioned European search report.
The members are as contained in the European Patent Office EDP file on
The European Patent Office is in no way liable for these particulars which are merely given for the purpose of information.

08-06-2026

| Patent document cited in search report | Publication date | Patent family member(s) | Publication date |
|---|---|---|---|
| ------------------------------------------------------------------------------------ | | | |

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GUO Y** ; **BIAN J** ; **LI Q** ; **LEAVITT T** ; **ROSENBERG EL et al.** A 3-minute test of cardiorespiratory fitness for use in primary care clinics. *PLOS ONE*, 2018, vol. 13 (7), e0201598